(19) Europäisches Patentamt
European Patent Office
Office européen des brevets

(11) Veröffentlichungsnummer: **0 508 258 A1**

(12) # EUROPÄISCHE PATENTANMELDUNG

(21) Anmeldenummer: **92105444.1**

(22) Anmeldetag: **30.03.92**

(30) Priorität: **12.04.91 DE 4111902**

(43) Veröffentlichungstag der Anmeldung:
**14.10.92 Patentblatt 92/42**

(84) Benannte Vertragsstaaten:
**CH DE FR GB IT LI NL**

(51) Int. Cl.5: **C07C 255/31**, C11B 9/00

(71) Anmelder: **Haarmann & Reimer GmbH**
**Postfach 1253**
**W-3450 Holzminden(DE)**

(72) Erfinder: **Hopp, Rudolf, Dr.**
**Auf dem Gehrenkamp 28**
**W-3450 Holzminden(DE)**
Erfinder: **Thielmann, Thomas, Dr.**
**Brüder-Grimm-Weg 19**
**W-3450 Holzminden(DE)**
Erfinder: **Göttsch, Wilhelm**
**Eichenhang 12**
**W-3454 Bevern(DE)**

(74) Vertreter: **Petrovicki, Wolfgang, Dr. et al**
**Bayer AG Konzernverwaltung RP Patente Konzern**
**W-5090 Leverkusen 1 Bayerwerk(DE)**

(54) **Cyclopentyl-cyanomethyl-cyclopentene, Verfahren zu ihrer Herstellung und ihre Verwendung als Riechstoffe.**

(57) Durch Knoevenagel-Kondensation von 2-Cyclopentyl-cyclopentanon und Cyanessigsäure und Decarboxylierung des Reaktionsprodukts werden neue Nitrile zugänglich, die Riechstoffe mit einer überaus frischen Note darstellen.

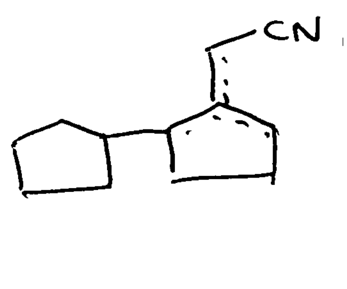

EP 0 508 258 A1

Die Erfindung betrifft neue Verbindungen der Formel

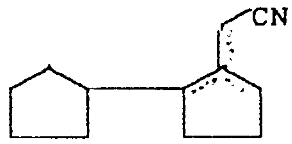

(I)

worin die gestrichelte Linie die drei möglichen Positionen einer C = C-Doppelbindung angibt, ein Verfahren zur Herstellung dieser Verbindungen durch Knoevenagel-Kondensation von 2-Cyclopentylcyclopentanon und Cyanessigsäure und Decarboxylierung des Reaktionsproduktes sowie die Verwendung der neuen Verbindungen als Riechstoffe.

Die Knoevenagel-Kondensation kann auf an sich bekannte Weise (vergl. z.B. Organikum, 15. Aufl., VEB Deutscher Verlag der Wissenschaften, Berlin, 1984, s. 571 ff.) erfolgen. Pro Mol 2-Cyclopentylcyclopentanon setzt man in der Regel 1 bis 3, vorzugsweise 1 bis 2 Mol Cyanessigsäure ein. Die Reaktion wird in Gegenwart von 0,005 bis 0,5, vorzugsweise 0,05 bis 0,3 Mol Katalysator pro Mol 2-Cyclopentylcyclopentanon, gegebenenfalls in Gegenwart von 0,1 bis 1, vorzugsweise 0,2 bis 0,5 Mol, Eisessig durchgeführt, wobei als Katalysatoren beispielsweise Piperidin, β-Alanin oder Ammoniumacetat in Frage kommen. Die Reaktion läßt sich am besten in einem organischen Lösungsmittel durchführen, das mit Wasser ein Aseotrop bildet. Geeignete organische Lösungsmittel dieser Art umfassen Aromaten wie Benzol, Toluol, Xylol; sie werden im allgemeinen in Mengen von 100 bis 1.000, vorzugsweise 100 bis 500 ml pro Mol 2-Cyclopentylcyclopentanon eingesetzt. Die Reaktionstemperatur wählt man am besten so, daß das entstehende Primärprodukt sofort decarboxyliert; vorzugsweise beträgt die Temperatur 100 bis 160, insbesondere 135 bis 145$^0$C. Sobald die Bildung von Wasser und Kohlendioxid beendet ist, kann das Reaktionsgemisch aufgearbeitet werden.

Die 3 möglichen Isomeren entstehen nicht alle in gleicher Menge:
Das Cyanomethylenderivat der Formel (II)

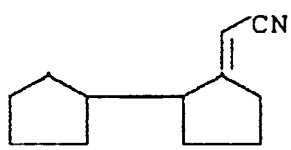

(II)

entsteht in untergeordneter Menge und ist geruchlich nicht von Interesse. Die bevorzugten erfindungsgemäßen Cyclopentyl-cyanomethylcyclopentene entsprechen der Formel (III)

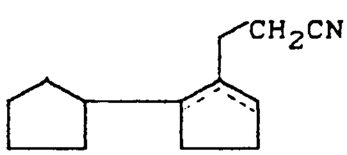

(III)

worin die gestrichelte Linie die beiden möglichen Positionen einer C = C-Doppelbindung angibt.

Auch die beiden Isomeren der Formeln (IV) und (V)

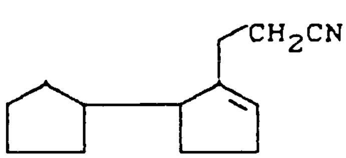

(IV)

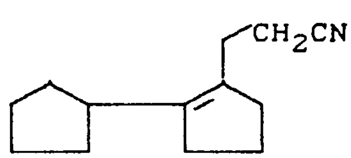

(V)

sind nicht gleichwertig, wie die folgenden Geruchsbeschreibungen zeigen:

Verbindung IV: aldehydisch, melonig grün;

Verbindung V: wäßrig, extrem starke Meeres-Algennote, etwas fruchtig nach Stachelbeeren oder Rhabarber.

Durch die Reaktionsbedingungen läßt sich das Verhältnis der entstehenden Isomeren beeinflussen (vergl. Beispiele 1 und 2). Die Abwesenheit von Eisessig und die Verwendung geringerer Lösungsmittelmengen (Beispiel 1) ergibt zwar eine größere Gesamtausbeute, aber eine geringere Ausbeute des erwünschten Produkts (V).

In Anwesenheit einer starken Base wie Alkalialkoholat (z.B. Natriumisopropylat) erfolgt eine teilweise Isomerisierung des weniger erwünschten Isomeren IV in das erwünschte Isomere V schon bei Raumtemperatur (vergl. Beispiel 3).

Die erfindungsgemäßen Verbindungen (I) können in Kombination mit anderen, an sich bekannten Riechstoffen (Arctander, Perfume and Flavor Chemicals, Montclair, N.J. (USA), 1969) und etherischen Ölen (Arctander, Perfume and Flavor Materials of Natural Origin, Elisabeth, N.J. (USA), 1960) angewendet werden und führen zu Parfumbasen und Riechstoffkompositionen mit ausdrucksstarken Noten, die sich hervorragend für die Parfümierung von Fertigprodukten des Aerosol-, des Waschmittel- und des Haushaltsreinigersektors, aber auch des Feinparfümerie- und des Kosmetiksektors eignen, z.B. für Detergentien, Haarpflegemittel, Badezusätze, Geschirrspülmittel, Waschpulver, Seifen, Antiperspirans, Puder, Cremes, Rasierwasser, Aftershave-Lotions, Raumluftverbesserer, WC-Reiniger, Sonnenschutzmittel.

Die isomeren Nitrile der Formel (I) können dabei einzeln oder als Mischung zum Einsatz gelangen.

In diesen Präparaten werden die erfindungsgemäßen Verbindungen I im allgemeinen in einer Menge von 0,001 bis 1 Gew.-%, vorzugsweise 0,01 bis 0,1 Gew.-%, bezogen auf das fertige Parfumöl, eingesetzt.

Die Herstellung der Parfumkompositionen und parfümierten Produkte kann in üblicher Weise, beispielsweise durch Zusammengeben der Komponenten, erfolgen.

Die Prozentangaben der nachfolgenden Beispiele bedeuten Gewichtsprozente.

## Beispiele

### Beispiel 1

In einem Dreihalskolben, der mit Rührer, Wasserabscheider und Gaszähluhr ausgestattet ist, werden 304 g (2 Mol) 2-Cyclopentylcyclopentanon, 205 g (2,4 Mol) Cyanessigsäure und 40 g (0,45 Mol) Ammoniumacetat in 300 g Xylol unter Rückfluß am Wasserabscheider erhitzt. Nach 6 Stunden haben sich 67 g einer wässrigen Phase sowie ca. 60 l $CO_2$ abgespalten. Das Reaktionsgemisch wird nacheinander mit 100 g 10 %iger Schwefelsäure, mit 200 g 5 %iger Natronlauge und mit Wasser gewaschen und über eine 30-cm Füllkörperkolonne destilliert. Man erhält neben 80 g 2-Cyclopentyl-cyclopentanon (T(Sumpf) = 130°C; T-(Kopf) = 85°C; 0,6 mbar) 180 g der erfindungsgemäßen Nitrile der Formel (I) (T(Sumpf) = 175°C; T(Kopf) = 100°C; 0,6 mbar). Bezogen auf umgesetztes Keton errechnet sich eine Ausbeute von 70 %.

Zusammensetzung der erhaltenen Nitrile laut Gaschromatogramm (Säule: 60M DB1; Temperaturbedingung: 80-4-250°C; d.h.: Gaschromatogramm temperaturprogrammiert, Anfangstemperatur: 80°C, kein isothermer Vorlauf; Aufheizrate: 4°C pro Minute; Endtemperatur: 250°C).

73,5 % 1-Cyanomethyl-5-cyclopentyl-cyclopent-1-en;
s. NMR-Spektrum, Fig.1.
21,2 % 1-Cyanomethyl-2-cyclopentyl-cyclopent-1-en;
s. NMR-Spektrum, Fig.2.
3,7 % 2-Cyanomethylen-dicyclopentyl

### Beispiel 2

In der in Beispiel 1 beschriebenen Apparatur werden 382 g (4,5 Mol) Cyanessigsäure, 456 g (3,0 Mol) 2-Cyclopentylcyclopentanon, 23 g Ammoniumacetat (0,3 Mol) und 72 g (1,2 Mol) Essigsäure in 900 ml Xylol unter Rückfluß am Wasserabscheider erhitzt. Nach 12 Stunden haben sich 101 l $CO_2$ sowie 74 g einer wässrigen Phase abgespalten. Das Reaktionsgemisch wird nacheinander mit 200 ml 10 %iger Schwefelsäure, mit 400 ml 5 %iger Natronlauge und mit Wasser gewaschen und über eine 30-cm Füllkörperkolonne destilliert. Man erhält neben 158 g 2-Cyclopentylcyclopentanon (T(Sumpf) = 130°C; T(Kopf) = 85; 0,6 mbar) 205 g der erfindungsgemäßen Nitrile der Formel (I) (T(Sumpf) = 175°C; T(Kopf) = 100°C; 0,6 mbar). Bezogen auf umgesetztes Keton errechnet sich eine Ausbeute von 60 %.

Zusammensetzung der erhaltenen Nitrile laut Gaschromatogramm (Säule: 60 DB 1; Temperaturbedingung: 80-4-250°C):

34,1 % 1-Cyanomethyl-5-cyclopentyl-cyclopent-1-en;

62,9 % 1-Cyanomethyl-2-cyclopentyl-cyclopent-1-en;
3,0 % 2-Cyanomethylen-dicyclopentyl

Beispiel 3

In einem Dreihalskolben, der mit Tropftrichter, Rückflußkühler und Stickstoffzuleitung ausgestattet ist, werden 0,5 g (0,021 Mol) Natrium in 100 g wasserfreiem Isopropanol unter Erhitzen gelöst. Die homogene Lösung wird auf Raumtemperatur abgekühlt und mit 100 g (0,57 Mol) einer Mischung der erfindungsgemäßen Nitrile der Formel (I) (enthaltend 62,8 % 1-Cyanomethyl-5-cyclopentyl-cyclopent-1-en;34,0 % 1-Cyanomethyl-2-cyclopentyl-cyclopent-1-en; 3,2 % 2-Cyanomethylen-dicyclopentyl versetzt und 4 Stunden bei Raumtemperatur nachgerührt. Im Anschluß wird mit Eisessig auf pH = 6 neutralisiert und destilliert.

Man erhält 85,0 g Destillat der Nitrile der Formel (I); Zusammensetzung siehe unten. Bezogen auf das eingesetzte Nitrilgemisch errechnet sich eine Ausbeute von 85 %. Die Rückstandsmenge beträgt 13 g.

Zusammensetzung des Destillates laut Gaschromatogramm (Säule: 60 m DB 1; Temperaturbedingung: 80-4-250°C):
8,8 % 1-Cyanomethyl-5-cyclopentyl-cyclopent-1-en;
66,0 % 1-Cyanomethyl-2-cyclopentyl-cyclopent-1-en;
25,2 % 2-Cyanomethylen-dicyclopentyl
Die in den folgenden Anwendungsbeispielen verwendeten Trivialnamen haben folgende Bedeutung:

Iraldein gamma rein:

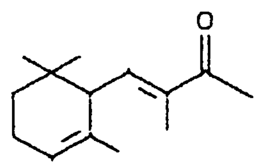

3-Methyl-4-(2.6.6-Trimethylcyclohex-2-en-1-yl)-but-3-en-2-on.

Eugenol

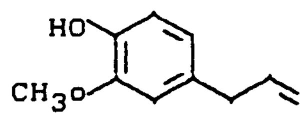

2-Methoxy-4-allylphenol

Moschus Keton

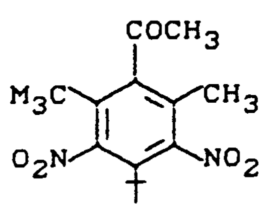

2.6-Dimethyl-3.5-dinitro-4-tert.-butylacetophenon.

Vertocitral: Mischung aus

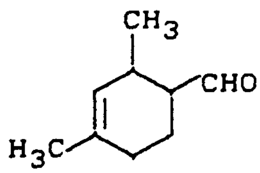

+

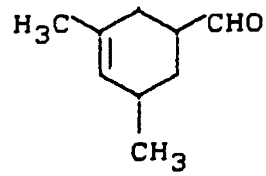

2.4-Dimethyl-cyclohex-3-en-1-aldehyd

3.5-Dimethyl-cyclohex-3-en-1-aldehyd

Evernyl:

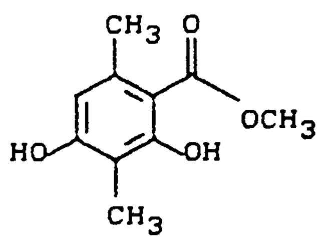

2.5-Dimethyl-4.6-dihydroxy-benzosäuremethylester

Anwendung 1

Eine Riechstoffkomposition wird durch Mischen folgender Bestandteile hergestellt (Mengenangabe in Gramm):

| | |
|---|---|
| Aldehyd C10 (Decanal) | 5,0 |
| Nitrile der Formel (I), 1 %ig in Dipropylenglykol (0,04 % in Parfumöl) | 40,0 |
| Galbanum resin | 20,0 |
| Bergamottöl dest. | 60,0 |
| Dihydromyrcenol | 50,0 |
| Dihydromyrcenylacetat | 40,0 |
| Citrylal (H + R) | 20,0 |
| Petitgrainöl franz. | 40,0 |
| Maiglöckchen-Base Muguet # 10689F (H + R) | 60,0 |
| Phenylethylalkohol rein | 60,0 |
| Citronellol supra | 50,0 |
| Benzylacetat | 50,0 |
| Hexylzimtaldehyd alpha | 60,0 |
| Hexenylsalicylat cis-3 | 100,0 |
| Benzylsalicylat | 80,0 |
| Iraldein gamma rein | 80,0 |
| Eugenol | 10,0 |
| Mousse C abs. verte jug. 50 % TEC | 10,0 |
| Moschus Keton | 80,0 |
| Dipropylenglykol | 85,0 |
| | 1.000,0 |
| TEC = Triethylcitrat | |

Die eingesetzten Nitrile der Formel (I) zeichnen sich dadurch aus, daß bereits bei Zugabe einer sehr geringen Menge zu einem geeigneten Parfumöl dieses sehr eindrucksvoll in Richtung "Meeresfrische" variiert wird.

Anwendung 2

Eine Riechstoffkomposition wird durch Mischen folgender Bestandteile hergestellt (Mengenangaben in Gramm):

| | |
|---|---|
| 2-Methyldodecanal | 1,0 |
| Nitrile der Formel (I), 1 %ig in Dipropylenglykol (0,02 % im Parfumöl) | 2,0 |
| Vertocitral (H + R) | 0,5 |
| Dihydromyrcenol | 5,0 |
| Terpinylacetat | 30,0 |
| Citrylal (H + R) | 4,0 |
| Orangenöl bras. | 6,0 |
| Spiköl span. | 6,0 |
| Eucalyptusöl globulus 80/85 % | 3,0 |
| Beifussöl marokk. | 1,0 |
| Isobornylacetat | 3,0 |
| Phenylethylalkohol rein | 3,0 |
| Geraniol | 6,0 |
| Benzylacetat | 4,0 |
| Hexylzimtaldehyd alpha | 3,0 |
| Nelkenblätteröl entfärbt | 1,0 |
| Cumarin | 3,0 |
| Cedernholzöl Florida | 6,0 |
| Evernyl (RBD) | 0,5 |
| Mousse A concr. 50 % in TEC | 1,0 |
| Moschus Keton | 2,0 |
| Indol 10 %-ig in Dipropylenglykol | 1,0 |
| Dipropylenglykol | 8,0 |
| | 100,0 |

Durch Zusatz der eingesetzten Nitrile der Formel (I) erhält die Kopfnote eine saubere Frische. Die Nitrile der Formel (I) besitzen einen sehr starken, frischen Meeresduft, der in Verdünnung an Algen, frischen Fisch und Meeresküste erinnert. Daneben finden sich krautig-würzige Noten von Eichenmoos, Dill und Cumin.

**Patentansprüche**

**1.** Verbindungen der Formel

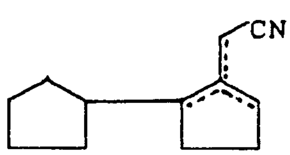

worin die gestrichelte Linie die drei möglichen Positionen einer C = C-Doppelbindung angibt.

**2.** Verbindung der Formel

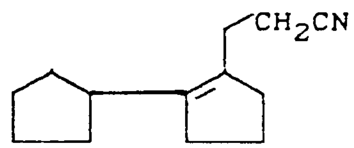

3. Verfahren zur Herstellung von Verbindungen nach Anspruch 1 durch Knoevenagel-Kondensation von 2-Cyclopentyl-cyclopentanon und Cyanessigsäure und Decarboxylierung des Kondensationsprodukts.

4. Verwendung der Verbindungen nach Anspruch 1 als Riechstoffe.

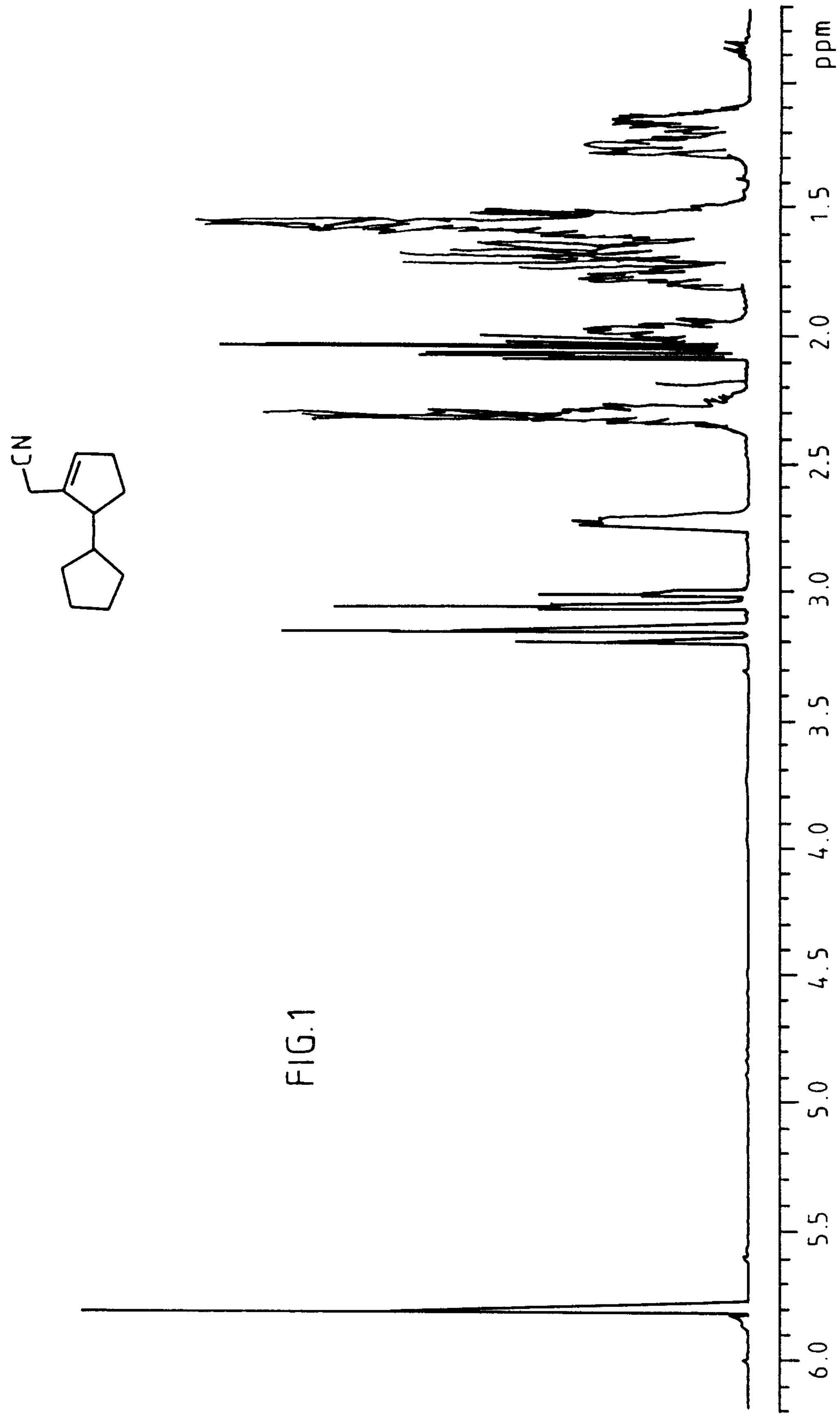


FIG.1

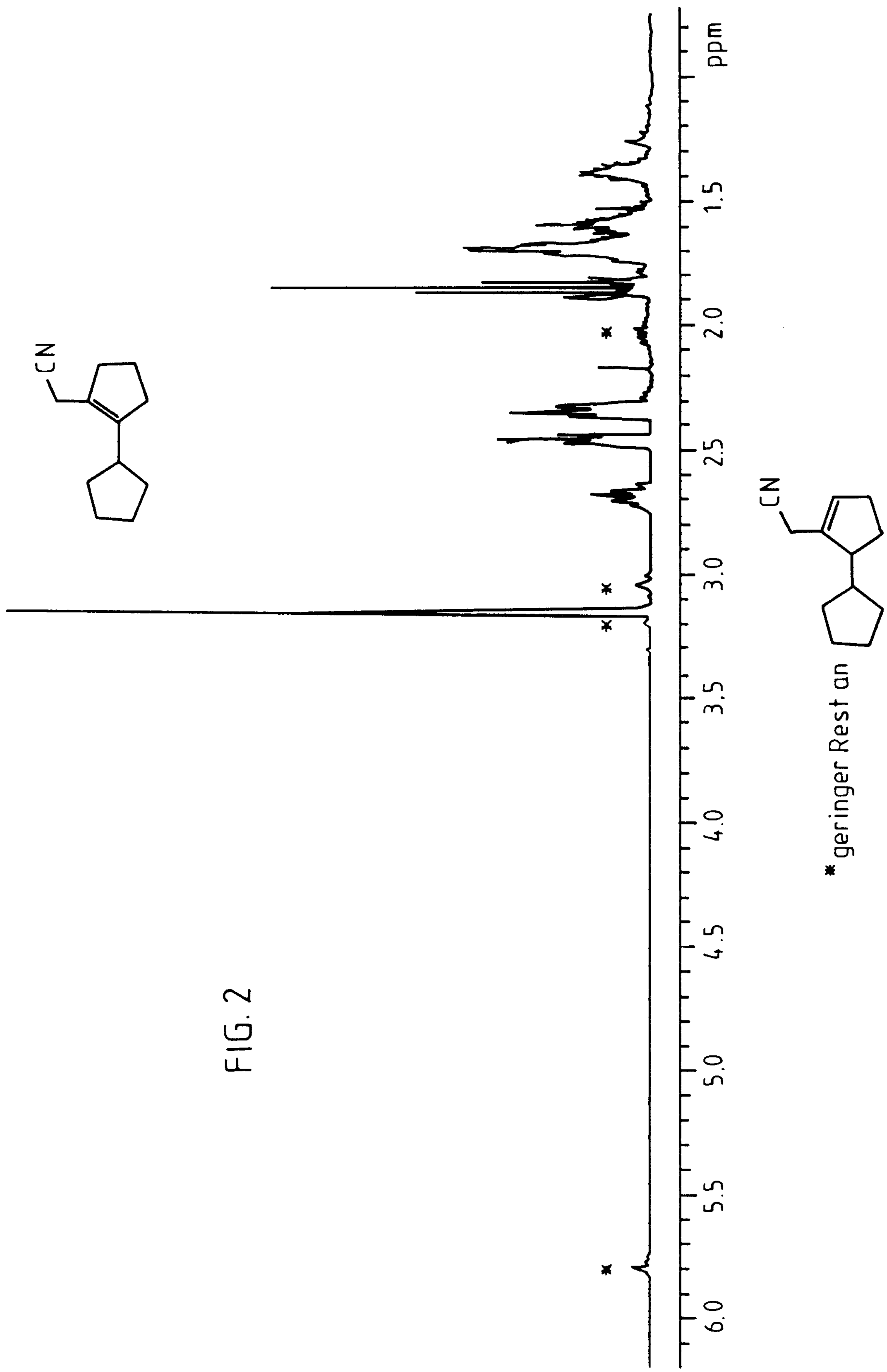


FIG. 2

Europäisches Patentamt

# EUROPÄISCHER RECHERCHENBERICHT

Nummer der Anmeldung

EP 92 10 5444

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 016 650 (NIPPON ZEON)<br>* Ansprüche 1,2 *<br>--- | 1,2,4 | C07C255/31<br>C11B9/00 |
| A | EP-A-0 302 816 (DRAGOCO)<br>* Ansprüche 1,2,3 *<br>--- | 1,2,4 | |
| A | EP-A-0 149 054 (CHEMISCHE WERKE HÜLS)<br>* Ansprüche 1,7 *<br>----- | 1,2,3,4 | |
| | | | RECHERCHIERTE SACHGEBIETE (Int. Cl.5 ) |
| | | | C07C<br>C11B |

Der vorliegende Recherchenbericht wurde für alle Patentansprüche erstellt

| Recherchenort | Abschlußdatum der Recherche | Prüfer |
|---|---|---|
| DEN HAAG | 09 JULI 1992 | HEYWOOD C.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument